# EUROPÄISCHE PATENTSCHRIFT

(11) **EP 1 132 385 B1**
(45) Veröffentlichungstag und Bekanntmachung des Hinweises auf die Patenterteilung: **15.10.2003**
(21) Anmeldenummer: 01710005.8
(22) Anmeldetag: 01.02.2001
(51) Int. Cl.: C07D 311/72

(54) **Verfahren zur Herstellung von alpha-Tocopherolacetat durch Kondensation von Trimethylhydrochinon-Diestern mit Isophytol**
Process for the production of alfa-tocopherolacetate by condensation of trimethylhydroquinone-diesters with isophytol
Procédé de production d'acétate d' alfa-tocophérol par condensation de diesters de triméthylhydroquinone avec l' isophytol

(30) Priorität: 09.03.2000 DE 10011402
(43) Veröffentlichungstag der Anmeldung: 12.09.2001
(73) Patentinhaber: Degussa AG, 40474 Düsseldorf (DE)
(72) Erfinder: Huthmacher, Klaus, Dr., 63584 Gelnhausen (DE); Kretz, Stephan, 63599 Biebergemünd (DE); Krill, Steffen, Dr., 67346 Speyer (DE)

(56) Entgegenhaltungen:
- EP-A- 0 850 937
- EP-A- 0 924 208

## Beschreibung

Die vorliegende Erfindung betrifft ein neues Kreislaufverfahren zur Herstellung von α-Tocopherol-Acetat durch Kondensation von Trimethylhydrochinon-Diestern und Isophytol in Gegenwart eines Katalysatorsystems bestehend aus einem Zinkhalogenid (Lewissäure) und einer wässrigen Protonensäure (Brönstedtsäure) und gegebenenfalls eines elementaren Metalls in einem mit Wasser extrahierbaren oder mischbaren polaren Lösungsmittel/Wasser Gemisch und anschließender Rückführung des Katalysatorsystems.

α-Tocopherol und seine Derivate sind als Futtermitteladditive, Antioxidanten, Mittel zur Anregung des Blutkreislaufs, Mittel zur Verringerung der Zellalterung und für verwandte Anwendungen von Bedeutung. Für Anwendungen als Futtermitteladditiv sind insbesondere pulverförmige Formulierungen des α-Tocopherol-Acetats (Vitamin E-Acetat) mit einer geeigneten Kieselsäure im Markt etabliert.

Bekannt sind vor allem Verfahren zur Herstellung von α-DL-Tocopherol, also der unveresterten, nicht lagerstabilen, licht-empfindlichen Vitamin E Form. Alpha-Tocopherol wird nach diesen Verfahren zunächst durch Kondensation von Trimethylhydrochinon mit Isophytol unter Kondensation von Wasser hergestellt und in einem separaten Schritt mit stöchiometrischen Mengen eines Acylierungsmittels.zum Vitamin E-Acetat verestert. Im folgenden Schema ist diese Vorgehensweise skizziert:

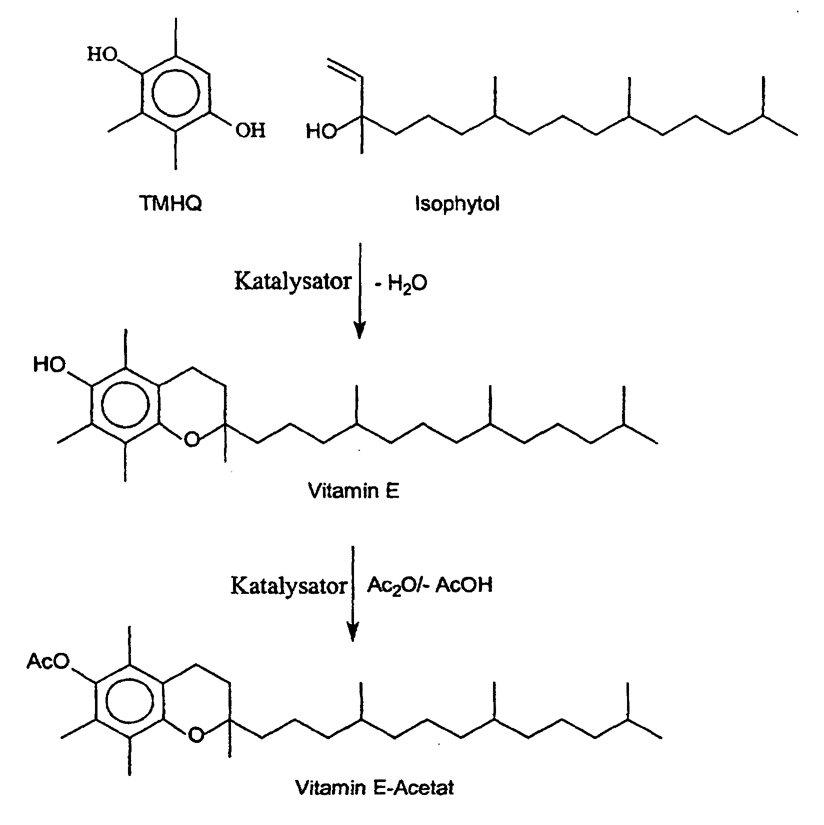

Gemäß diesem Stand der Technik geht man im allgemeinen von Trimethylhydrochinon (TMHQ) aus, das unter Verwendung verschiedener Katalysatorsysteme mit Isophytol umgesetzt wird. (DE-OS 4243464 (= US PS 5,523,420,) DE-OS 19603142, EP 0694 541, DE 196 03 142, EP (0 949 255 A1).

Nach beendeter Reaktion muß das Produkt anschließend vollständig acetyliert werden, um z. B. zum lagerstabilen, handelsüblichen Vitamin-E-Acetat zu gelangen. All diesen Verfahren ist gemeinsam, daß sie nicht direkt zu Tocopherolacetat, der lagerstabilen, handelsüblichen Vitamin E Form gelangen.

Es hat auch Versuche gegeben, Trimethylhydrochinon-Ester als Edukt der Synthese von Tocopherolen mit Isophytol umzusetzen.

Der Einsatz der entsprechenden Trimethylhydrochinonester, beispielsweise Trimethylhydrochinon-Diacetat (TMHQ-DA) als Syntheseäquivalent von TMHQ, hat den prinzipiellen Vorteil, daß die im Endprodukt Vitamin E-Acetat vorhandene Acetylgruppe bereits im Eduktmolekül TMHQ-DA vorhanden ist und man durch geschickte Reaktionsführung durch Kondensation von TMHQ-DA mit Isophytol direkt zum Vitamin E-Acetat gelangt ohne die Notwendigkeit, in einem separaten Reaktionsschritt mit stöchiometrischen oder sogar überstöchiometrischen Mengen Acetanhydrid nachträglich verestern zu müssen. Bislang konnte jedoch kein wirtschaftliches Verfahren gefunden werden, das es erlaubt, die Kondensation der als Edukt verwendeten Trimethylhydrochinon-Dioder Mono-Ester mit Isophytol mit ausreichenden Ausbeuten durchzuführen und direkt zu Vitamin E-Acetat zu gelangen.

FR-A 2 259 822 (DE-OS 2 404 621) betrifft den Einsatz von diacetyliertem Trimethylhydrochinon TMHQ-DA. Die dort beschriebene Kondensation mit Isophytol in Gegenwart einer festen Säure liefert jedoch nur eine Ausbeute von ca. 41% α-DL-Tocopherol (Vitamin E) und führt nicht zu den entsprechenden Estern.

Die Kondensation von Trimethylhydrochinonmonoacetat (TMHQ-MA) mit Isophytol wird in der DE-OS 2 160 103 ( = US-PS 3,789,086) beschrieben. Man erhält unter Verwendung von Fe(II)Cl₂ und Salzsäure bei gleichzeitiger Abtrennung des Reaktionswassers nur geringe Mengen des α-Tocopherol-Acetats und muß unter Aminkatalyse und Zugabe überstöchiometrischer Mengen an Acetanhydrid nachacetylieren. Die Ausbeuten sind ungenügend, die zweistufige Verfahrensweise ist aufwendig und die Notwendigkeit, mit überstöchiometrischen Mengen Acetanhydrid zum Vitamin E-Acetat umzusetzen hat einen hohen Chemikalienverbrauch zur Folge. Auf die Aufarbeitung der nach der Reaktion anfallenden Katalysatorphase wird nicht eingegangen.

Gemäß der JP-OS 51-80859 (15. Juli 1976) setzt man Trimethylhydrochinon oder dessen Ester mit Isophytol in Gegenwart von Zinkchlorid um. Die Reaktion führt bei Temperaturen von mehr als 100°C entsprechend der Aufgabenstellung zum α-Tocopherol.

Nachteilig an diesen bezüglich der erreichten Ausbeuten recht wirtschaftlichen Verfahren ist die durch die Verwendung von großen Zinkchloridmengen auftretende Abwasserproblematik. Eine einfache Recyclierung dieser nach Extraktion anfallenden wässrigen Zinkchloridlösungen ist nicht möglich, da im Falle der Kondensation von TMHQ bei der Umsetzung zusätzlich zum für die Extraktion benötigten Wasser noch Reaktionswasser gebildet wird, das die Katalysatorlösung desaktiviert (siehe Bull. Chem. Soc. Japan., 68, (1995), 3569 ff und Bull. Chem. Soc. Japan., 69 ,(1996), 137. Versuche, die mit Wasser extrahierte Zinkhalogenid Phase (ca. 20-60 Gew.-% ZnCl₂) zu recyclieren und wieder für die Kondensation einzusetzen, resultieren in einer Verringerung der Reaktionsausbeute und einer schlechteren Produktqualität. In der Patentanmeldung EP 0 850 937 A1 wird die Umsetzung in einem mit Wasser nicht oder nur wenig mischbaren Lösungsmittel durchgeführt, nach der Reaktion die Katalysatorphase mit Wasser extrahiert und nach Aufkonzentrierung der wässrigen Phase auf etwa 60-90% die so erhaltene Katalysatorlösung bei 20-200°C in die Reaktion zurückgeführt. Nachteilig an dieser Verfahrensweise ist die Tatsache, daß die Zinkhalogenidmischungen bei Raumtemperatur als Maische vorliegt und deshalb nur durch spezielle für diesen Anwendungsbereich vorgesehene Pumpen gefördert werden kann. Um zu einer flüssigen Katalysatorform zu gelangen, muß die Maische auf eine entsprechende Temperatur begleitbeheizt werden, was ebenfalls einen erheblichen Aufwand erfordert und dadurch unwirtschaftlich ist.

Ein erheblicher Nachteil an diesem Verfahren ist hohe Flüchtigkeit der zur Reaktion notwendigen Protonensäure, insbesondere HCl bei der destillativen Wasserentfernung zur Aufkonzentrierung der Zn-Chlorid-Lösungen. Durch diesen Verlust muss jeweils beim nächsten Ansatz die HCl Menge zudosiert werden, was das Verfahren kompliziert.

Das bislang bezüglich der Ausbeuten zur Umsetzung von TMHQ-DA effizienteste Verfahren wird in der DE-OS 197 57 124.7 beschrieben, bei dem ein binäres Katalysatorsystem aus einem Zinkhalogenid einerseits und einer Protonensäure andererseits in diversen inerten, aprotischen Lösungsmitteln wie beispielsweise Essigsäureester oder Aromaten (Toluol) verwendet wird. Die Aufarbeitung erfolgt hier durch wässrige Extraktion der gesamten Katalysatorphase nach der Kondensation, wobei in der Produktphase ein Gemisch von α-Tocopherol neben α-Tocopherol-Acetat vorliegt. Bei dieser Verfahrensweise muß zur nachträglichen Veresterung des α-Tocopherols zusätzlich zur Acetylierung ein Katalysator zugegeben werden, der nach der Reaktion ebenfalls durch wässrige Extraktion wieder entfernt werden muß. Bei Verwendung von Ester-haltigen Lösungsmitteln ergibt sich eine weitere Schwierigkeit durch die Anwesenheit von Wasser während der Reaktion, so daß es neben der in situ stattfindenden Verseifung des TMHQ-DA auch zur teilweisen Verseifung des Lösungsmittels kommt. Auf diese Weise entstehen aus dem als Lösungsmittel eingesetzten Ester die entsprechenden organischen Säuren und Alkohole, die über aufwendige Trennverfahren vom Produkt entfernt werden müssen oder sich bei Rückführung des Lösungsmittels im Kreislaufverfahren anreichern.

Die Herstellung von 2,3,5-Trimethylhydrochinon-Diestern erfolgt in bekannter Weise aus Ketoisophoron (4-Oxo-Isophoron = KIP) in Gegenwart eines sauren Katalysators und eines Acylierungsmittels wie Carbonsäureanhydride (im einfachsten Fall Acetanhydrid unter Abspaltung von Essigsäure) oder Acylhalogenide. Die entsprechenden Verfahren sind in mehreren Patentschriften dokumentiert (z.B. DE 2 149 159, EP 0 808 815 A2, EP 0 850 910 A1, EP 0 916 642 A1). Die Bildung des gebräuchlicherweise für die Vitamin E-Acetat Synthese verwendeten TMHQ-DA erfolgt in allen Fällen in Gegenwart eines sauren Katalysators oder einem Gemisch aus mehreren geeigneten Säuren unter entsprechenden Bedingungen. Man erhält üblicherweise nach Reaktionsende ein Gemisch aus TMHQ-DA, einigen aromatischen Nebenprodukten, im wesentlichen Trimethyl-Brenzcatechin-Diester und Trimethylphenol-Derivate, überschüssigem Acylierungsmittel, dem Katalysator und Essigsäure. Dieses Gemisch muß zur Abtrennung des als Edukt für die Kondensation mit Isophytol verwendeten TMHQ-DA aufgearbeitet werden, wobei man im einfachsten Fall durch Kristallisation aus dieser essigsauren Lösung eine ausreichende TMHQ-DA Qualität erhält. Das nach dieser Verfahrensweise isolierte Produkt enthält je nach Reinigungsgrad noch einen Essigsäuregehalt bis zu 50 Gew.-%.

Bei den im obigen beschriebenen, den Stand der Technik representierenden Verfahren, wird nicht auf die Aufarbeitung der bei der Reaktion verwendeten Katalysatorlösungen eingegangen.

Aufgabe der Erfindung ist es, ein verbessertes Verfahren zur Herstellung von α- Tocopherolestern im Kreislaufverfahren zur Verfügung zu stellen, bei dem die Reaktion schon im ersten Schritt möglichst weit in Richtung des Esters abläuft und die bei der Reaktion nach Aufarbeitung erhaltene Katalysatorphase so zu regenerieren, daß sie auf einfache Weise erneut in die Reaktion ohne Erniedrigung der katalytischen Aktivität zurückgeführt werden kann. Insbesondere war es Aufgabe der Erfindung, ein Verfahren zu finden, daß es ermöglicht, die aktive Katalysatorlösung in Form einer auch bei Raumtemperatur gut handhabbaren, leicht dosierbaren (flüssigen) Form zur recyclieren, ohne daß es durch die Wiederverwendung der Katalysatorlösung zu geringeren Ausbeuten oder einer Verschlechterung der Produktqualität kommt.

In diesem Zusammenhang sollte auch ein Verfahren gefunden werden, das es erlaubt, die eingesetzten Katalysatorkomponenten, insbesondere auch die Protonensäure, nach der Katalysatorregenerierung zurückzuführen, und so die Notwendigkeit der kompletten Frischergänzung dieser Komponente zu umgehen.

Es war eine weitere Aufgabe der Erfindung, ein Verfahren zu finden, bei dem auch ein essigsaures TMHQ-DA, wie es bei der Synthese ausgehend von KIP als Rohprodukt erhalten wird, eingesetzt werden kann.

Die Erfindung betrifft ein Verfahren zur Herstellung von alpha-Tocopherolacetat durch Kondensation von Trimethylhydrochinon-Diacetat (TMHQ-DA) und Isophytol (IP) bei moderaten Temperaturen in Gegenwart eines Katalysatorsystems bestehend aus einem Zinkhalogenid und einer Protonensäure und gegebenenfalls einem elementarem Metall, insbesondere Zink, in Essigsäure als Lösungsmittel, wobei nach der Kondensationsreaktion eine Nachacetylierung-des nach der Kondensation erhaltenen Gemischs aus Tocopherol/Tocopherol-Acetat bei moderaten Temperaturen in Gegenwart des Kondensationskatalysators durchgeführt wird, das nach Abtrennung der essigsauren Katalysatorphase nach der Kondensation in der organischen Phase verbleibt, unter Regenerierung und Rückführung einer wässrigen, essigsauren Katalysatorlösung. Als Zinkhalogenidkatalysator werden insbesondere die Chloride und Bromide, aber auch Mischungen dieser Komponenten eingesetzt. Auch die basischen Chloride und Bromide des Zinks, also die entsprechenden Oxy- und Hydroxyhalogenide stellen aktive Katalysatoren des erfindungsgemäßen Verfahrens dar.

Durch die Verwendung von TMHQ-DA als Äquivalent von TMHQ und dessen direkte Umsetzung mit Isophytol wird Vitamin E-Acetat erhalten. Eine äquimolare Nachacetylierung ist nicht notwendig, sondern nur eine partielle Nachacetylierung, da im nach der Reaktion erhaltenen Produktgemisch nur noch wenig Vitamin E neben der Hauptmenge Vitamin E-Acetat vorhanden ist.

In diesem Zusammenhang gestattet die Verwendung von Essigsäure als Lösungsmittel, Vitamin E-Acetat in Roh-Ausbeuten von >96% zu erhalten, wobei als Aromatenkomponente neben isoliertem TMHQ-DA auch essigsaures TMHQ-DA (aus der TMHQ-DA Synthese ex KIP als Rohprodukt zugänglich) eingesetzt werden kann.

Die Hauptmenge des Katalysators kann nach der Kondensationsreaktion durch einfache Phasentrennung der essigsauren Phase (Katalysatorphase I) von der Vitamin E/Vitamin E-Acetat Phase (Produktphase I) getrennt werden, wobei in der organischen Phase noch eine ausreichende Katalysatorkonzentration verbleibt, um eine schonende, hoch selektive Nachacetylierung bei moderaten Temperaturen zu gestatten. Nach der Acetylierung wird die Vitamin E-Acetat Phase durch wässrige Extraktion von den Katalysatorresten befreit und die erhaltene wässrige Katalysatorphase (Katalysatorphase III) mit der nach der Kondensation erhaltenen Katalysatorphase I (II) vereinigt. Die Aufarbeitung dieser Katalysatorphasen erfolgt im einfachsten Fall durch destillative Abtrennung eines Gemisches aus Essigsäure und Wasser ohne daß die aktiven Katalysatorkomponenten im Destillat mit übergehen. Es verbleibt eine essigsaure, wässrige konzentrierte Katalysatorlösung, die Katalysatorphase IV, die wieder für die Kondensation eingesetzt werden kann.

Diese Katalysatorlösung ist auch bei Raumtemperatur flüssig und stellt eine ideale, bei moderaten Temperaturen einfach handhabbare und dosierbare Formulierung des aktiven Katalysators dar.

Die Verwendung von Essigsäure als bevorzugtes Lösungs- und Extraktionsmittel für die Katalysatorlösung ermöglicht die Recyclierung der Katalysatorlösung in Form einer gut handhabbaren, wässrig, essigsauren Lösung, die durch einfache Destillation von Essigsäure und Wasser so regeneriert werden kann, daß keine der katalytisch wirksamen Komponenten mit dem Destillat verloren gehen und die erhaltene Katalysatorlösung ohne Einbuße der Aktivität wieder in die Reaktion zurückgeführt werden kann.

Im vereinfachten Blockfließbild stellt sich eine Ausführungsform des Verfahrens wie folgt dar:

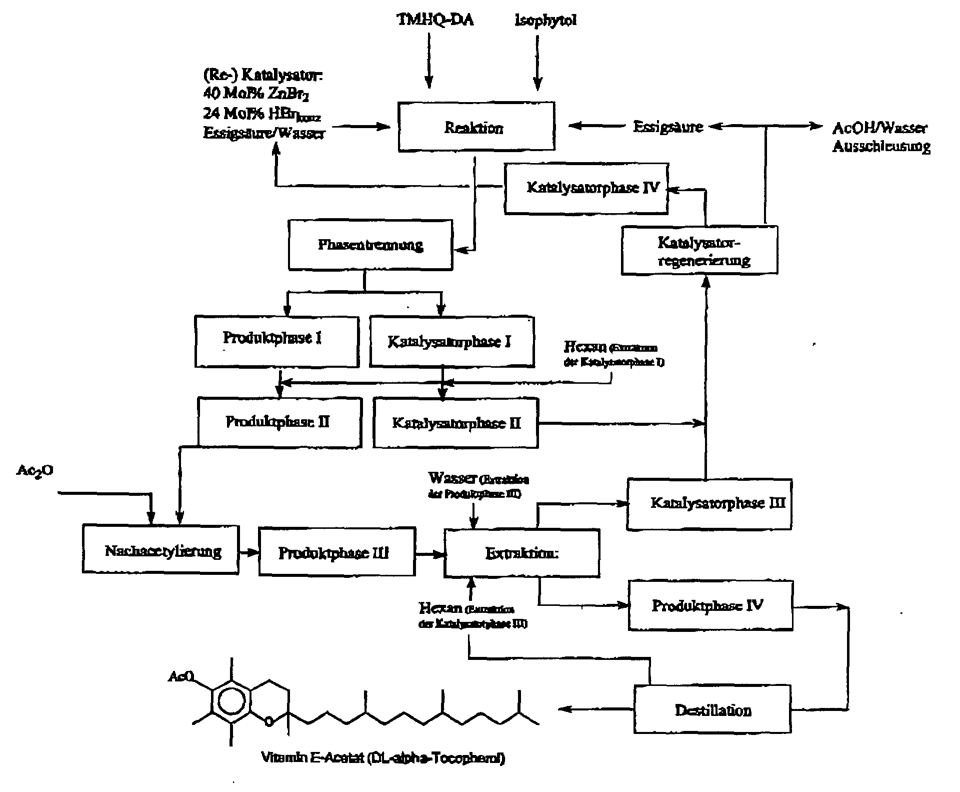

Die Kondensation des Aromatenbausteines TMHQ-DA mit Isophytol in Gegenwart des Katalysatorsystems aus ZnX₂ und HY (X = Halogenid-, Hydroxid-, Oxid-; Y = Anion einer Brönstedtsäure) und gegebenenfalls einem als dritte Katalysatorkomponente zugesetzten elementarem Metall, insbesondere Zink, verläuft in hervorragenden Ausbeuten, wenn man die Umsetzung allgemein in einem mit Wasser extrahierbaren oder mischbaren, protischen Lösungsmittel, insbesondere Essigsäure, durchführt und die zur Kondensation und nachfolgenden Acetylierung verwendete Katalysatorlösung in Form einer wässrigen, essigsauren Lösung von ZnX₂ und HY insbesondere Chlor und Bromwasserstoffsäure, in die Reaktion einführt.

Die Umsetzung der als Edukt verwendeten Komponenten in Essigsäure ergibt hervorragende Ausbeuten. Essigsäure hat gegenüber den üblicherweise zur Kondensation als Lösungsmittel eingesetzten Estern den Vorteil, daß sie sich unter den Reaktionsbedingungen inert verhält, während entsprechende Ester in Anwesenheit der sauren Katalysatoren und Wasser zur Hydrolyse neigen.

An dieser Stelle unterscheidet sich das Verfahren zum Einsatz von TMHQ-Diestern als Syntheseäquivalent von TMHQ wesentlich, da üblicherweise (beim TMHQ Verfahren) das Wasser als Azeotrop mit dem Lösungsmittel während der Reaktion entfernt wird und so keine Reaktion mit als Lösungsmittel verwendeten Estern eingehen kann. Beim Einsatz von TMHQ-DA muß jedoch in situ eine Verseifung des Diesters stattfinden, daß intermediar der entsprechende Monoester des TMHQ gebildet werden kann, denn nur die phenolische Hydroxylfunktion steht für die Kondensation mit Isophytol als reaktive Gruppe zur Verfügung.

Die Essigsäure kann für jeden Ansatz frisch zugegeben werden. In einer bevorzugten Variante wird als Lösungsmittel die in einem ersten Ansatz bei der Acetylierung mit Acetanhydrid als Nebenprodukt gewonnene Essigsäure verwendet. Ein weiterer Teil der Essigsäure gelangt durch die Verwendung von rohem, ungetrockneten TMHQ-DA aus der TMHQ-DA Herstellung ex KIP und Acetanhydrid in die Reaktion.

Die Essigsäurekonzentration kann bezüglich dem eingesetzten TMHQ-DA ca. 10-300 Gew.-% betragen, wobei üblicherweise die besten Ergebnisse bei 50 bis 150 Gew.-% Essigsäure bezüglich TMHQ-DA erreicht werden.

Die Wassermenge wird in der Reaktionsmischung auf eine Konzentration von 10⁻² - 200 Mol% bezüglich TMHQ-DA eingestellt, wobei sich die Wassermenge summarisch aus dem Gehalt der recyclierten wässrigen, essigsauren Katalysatorphase und der frisch ergänzten wässrigen HY (Katalysator-Protonensäure) ergibt. Im wesentlichen wird die Wasserkonzentration in der Reaktionsmischung durch den Wassergehalt der recyclierten Katalysatorphase bestimmt.

Die Kondensationsreaktion wird in Anwesenheit der Katalysatorkomponenten ZnX₂- HY und gegebenfalls eines elementaren Metalls in Essigsäure als Lösungsmittel bei Temperaturen zwischen 0°C - 150°C durchgeführt, wobei die besten Ergebnisse in einem Temperaturintervall von 40°C - 80°C erzielt werden. Die anschließende Acetylierung wird in Gegenwart der Katalysatorkomponenten ZnX₂ - HY und gegebenfalls eines elementaren Metalls bei Temperaturen zwischen - 20°C - 100°C durchgeführt, wobei die besten Ergebnisse bei 0°C - 40°C erzielt werden.

Als Lewissäure sind gemäß Patentliteratur DE 197 57 124 A1, Zinksalze, insbesondere die Halogenide wie Zinkchlorid und Zinkbromid geeignet, wobei auch die entsprechenden unter Reaktionsbedingungen entstehende Hydroxide in diese Terminologie miteinbezogen sind. Die Einsatzmengen der Lewissäuren betragen bezogen auf das eingesetzte TMHQ-DA 10 Mol% - 200 Mol%, insbesondere 20 Mol% - 50 Mol%. Im wesentlichen wird im Kreislaufverfahren bei Recyclierung der regenerierten Katalysatorlösungen die Lewissäurekonzentration über den Lewissäuregehalt der wässrigen, essigsauren Recyclierlösung eingestellt.

Die Lewissäure muß nicht als käufliche Komponente in die Reaktion eingebracht werden, sondern kann in situ durch Mischung entsprechender Mengen Halogenwasserstoffsäure mit dem entsprechenden Metall, insbesondere Zink hergestellt werden. Nach Regenerierung der Katalysatorlösung kann das entsprechende Zinkhalogenid nahezu vollständig wieder detektiert werden, fehlende Mengen werden durch Frischergänzung des elementaren Metalls und einer wässrigen Halogenwasserstoffsäure bis auf das gewünschte Konzentrationsniveau ausgeglichen.

Als Protonensäure sind gemäß Patentliteratur DE 197 57 124 A1 Halogenwasserstoffsäuren in konzentrierter Form oder in Form ihrer wässrigen Lösungen einsetzbar. Zu guten Ergebnissen gelangt man insbesondere bei Verwendung von Bromwasserstoff. Als Säuren sind aber auch Schwefelsäuren, Schwefelsäure/SO₃ Mischungen mit verschiedenen SO₃ -Konzentrationen und Supersäuren mit einem H₀-Wert kleiner gleich - 11,9, wie beispielsweise die Perfluoralkansäuren, oder auch Gemische von Borsäure und Oxalsäure geeignet. Die Einsatzmengen der Protonensäuren betragen bezogen auf das eingesetzte TMHQ-DA 0,01 Mol% - 100 Mol%, insbesondere 5 Mol% - 50 Mol%.

Im wesentlichen wird im Kreislaufverfahren bei Recyclierung der regenerierten Katalysatorlösungen die Protonensäurekonzentration über den Protonensäuregehalt der wässrigen, essigsauren Recyclierlösung eingestellt.

Die Einsatzmengen des verwendeten elementaren Metalls betragen bezogen auf das eingesetzte TMHQ-DA 0,01 Mol% - 100 Mol%, insbesondere 1 Mol% - 50 Mol%.

Die Reihenfolge der Edukt-/Katalysatorzugabe ist bis auf Isophytol beliebig, das zum Schluß zur Mischung der übrigen Komponenten gegeben wird.

In einer bevorzugten Ausführungsform legt man beim Anfahren die als Lösungsmittel verwendete Essigsäure vor (z.B. Essigsäure aus TMHQ-DA Herstellung, oder aus vorigem Ansatz der Vitamin E-Acetat Herstellung nach der Acylierung mit Acetanhydrid, oder als Frisch Lösungsmittel) und löst darin die Katalysatorkomponenten, die wässrige Halogenwasserstoffsäure und das ensprechende Zinkhalogenid und gegebenenfalls elementares Zink, auf. Zu dieser Lösung gibt man TMHQ-DA. Die so erhaltene Suspension wird auf Reaktionstemperatur (ca. 60°C) gebracht. Zu dieser Mischung wird während 2-6 Stunden Isophytol dosiert, gegebenenfalls als Essigsäurelösung. Nach Reaktionsende wird die Reaktionsmischung auf Raumtemperatur abgekühlt, wobei sich zwei definierte Phasen bilden, die Katalysatorphase (Katalysatorphase I) und die Produktphase (Produktphase I).

Die untere, schwere Phase enthält Vitamin E/Vitamin E-Acetat als Nebenkomponente und besteht hauptsächlich aus einer wässrigen, essigsauren Lösung der Katalysatorkomponenten. Der Anteil an Produktkomponenten (Vitamin E und Vitamin E-Acetat) in der Katalysatorphase I beträgt etwa 0,1 - 5 Mol%, üblicherweise 0,5 - 2 Mol% der insgesamt gebildeten Produktmenge. Die in der Katalysatorphase enthaltenen Produktanteile können durch einfache Extraktion mit einem geeigneten Lösungsmittel zurückgewonnen werden und. werden anschließend mit der oberen Produktphase vereinigt. Durch geeignete Reaktionsführung ist die Produktmenge in der Katalysatorphase so gering, daß auf eine Extraktion verzichtet werden kann.

Die obere Phase (Produktphase I) enthält Reste der Katalysatorkomponenten ZnX₂ und HY und als Hauptbestandteil ein Gemisch aus Vitamin E und Vitamin E-Acetat. Je nach Reaktionsführung schwankt das Verhältnis zwischen Vitamin E und Vitamin E-Acetat im Bereich zwischen 1:1 und 1:10, üblicherweise beträgt das nach der Kondensation erhaltene Verhältnis ca. 1:3. Als bestimmende Parameter für das Verhältnis zwischen Vitamin E und Vitamin E-Acetat kann vor allem die Wasserkonzentration in der Reaktionslösung und die Temperatur der Umsetzung identifiziert werden.

Die in der oberen Produktphase verbleibende Katalysatormenge ist ausreichend für die Acetylierung der neben Vitamin E-Acetat vorhandenen nicht veresterten Vitamin E Menge bei moderaten Temperaturen.

Nach der Phasentrennung von Katalysatorphase I von der Produktphase I wird der Anteil der Produktkomponenten, die etwa 0,1 -5 Mol% der insgesamt gebildeten Produktmenge ausmachen, aus der Katalysatorphase extraktiv entfernt. Als Extraktionsmittel können an dieser Stelle alle geeigneten, nicht oder nur wenig mit der Katalysatorphase mischbaren Lösungsmittel verwendet werden, insbesondere aliphatische, cycloaliphatische oder aromatische Lösungsmittel. Exemplarisch seien Pentan, Hexan, Heptan, Octan, Nonan, Decalin, Ligroin, Petrolether, Cyclohexan, Benzol, Toluol, Xylol, oder auch halogenierte Derivate der genannten Lösungsmittel genannt. Auch andere gebräuchliche Lösungsmittel wie Ester, insbesondere Carbonatester und aliphatische Carbonsäureester, und aliphatische Alkohole sowie Gemische aus den genannten Gruppen der Lösungsmittel sind für diese Extraktion geeignet.

Die Extraktion verläuft sehr effizient bereits mit geringen Mengen an aliphatischem Extraktionsmittel, wobei die Menge des Extraktionsmittels in Bereichen zwischen 1 Gew.-%-100 Gew.-% bezogen auf die zu extrahierende Katalysatorphase I variiert werden kann.

Die Extraktionsphase, die im wesentlichen aus Vitamin E/Vitamin E-Acetat und dem Extraktionsmittel besteht, wird mit der Produktphase I vereinigt, so daß insgesamt eine Phase, die Produktphase II resultiert, die sich additiv zusammensetzt aus der Produktphase I, die den Großteil des gebildeten Vitamin E und Vitamin E-Acetat enthält. In dieser Phase befinden sich 95-99,1% des insgesamt nach der Kondensationsreaktion gebildeten Vitamin E + Vitamin E-Acetat der Extrakt der Katalysatorphase I, der 0,1 -5 % des insgesamt gebildeten Vitamin E neben Vitamin E-Acetat enthält.

In einer weiteren ebenfalls erfindungsgemäßen Ausführungsform kann die Extraktion der Katalysatorphase I umgangen werden, indem vor der Phasentrennung das Extraktionsmittel zur nach der Kondensation erhaltenen Reaktionslösung gegeben wird. Auf diese Weise gelingt es, die nach der Phasentrennung erhaltene Produktphase I nahezu vollständig vom Wasser zu befreien, das die anschließende Acylierung stören würde. Die Acylierung wird dann unter geeigneten Bedingungen im Extraktionsmittel vorgenommen, ohne daß Reaktionsgeschwindigkeit und Selektivität der Reaktion wesentlich beeinflußt werden.

Ein weiterer Vorteil der Zugabe des Extraktionsmittels vor der ersten Phasentrennung ist die Tatsache, daß in der nach Phasentrennung erhaltenen Katalysatorphase I nur noch unwesentliche Mengen an Vitamin E und Vitamin E Acetat vorhanderi sind. Auf diese Art und Weise kann so die Extraktion der Katalysatorphase I, um diese Restmengen Wertstoff zu erhalten, eingespart werden.

Die Nachacetylierung kann batchweise oder kontinuierlich durchgeführt werden, wobei die Produktphase II sich zusammensetzt aus Essigsäure, dem Extraktionsmittel, Vitamin E neben Vitamin E-Acetat. Die in der zu acylierenden Phase vorhandene Rest-Wasserkonzentration wird gegebenenfalls durch Zugabe eines entsprechenden Überschusses an Acetanhydrid zerstört, wobei Essigsäure entsteht, die sowieso im Reaktionssystem von Anfang an anwesend ist. Die Produktphase II wird in einer vorteilhaften Ausführungsform mit Acetanhydrid versetzt, wobei die Reaktion durch die Anwesenheit des Katalysatorsystems aus Protonensäure/Lewissäure auch bei Raumtemperatur effizient katalysiert wird. Je nach Reaktionsführung und Konzentration der Katalysatorkomponenten kann die Reaktion in einem Temperaturintervall zwischen - 20°C - 100°C, bevorzugt zwischen 0°C - 40°C stattfinden.

Nach Ablauf der Reaktion resultiert die Produktphase III, die Vitamin E nur noch in einer Konzentration < 1% bezüglich Vitamin E-Acetat enthält. Zur Aufarbeitung dieser Produktphase wird in einem anschließenden Schritt eine Katalysator-Extraktion mit Wasser und gegebenenfalls einem Cosolvens, insbesondere Methanol oder Ethanol durchgeführt, wobei zur Unterstützung der Phasentrennung gleichzeitig ein mit Wasser nicht oder nur wenig mischbares Lösungsmittel eingesetzt wird, um die so erhaltene wässrige, essigsaure Katalysatorphase II von Produktresten zu befreien.

Für die Auswahl des Extraktionsmittels der organischen Produktphase III bzw. der Katalysatorphase III gelten die gleichen Kriterien, wie sie bereits im Falle der Extraktion der Katalysatorphase I angeführt wurden. Besonders bevorzugt ist eine Verfahrensweise, wobei man die Extraktion der Katalysatorphase I und die Extraktion der Katalysatorphase II im gleichen Extraktionsmittel durchführt. Besonders vorteilhaft ist die Durchführung dieser Trennung von Produkt (Vitamin E-Acetat) und Katalysator (ZnX₂- HY) als (gegebenenfalls) mehrstufige Gegenstromextraktion.

Man erhält nach Extraktion der Produktphase III mit Wasser und gegebenenfalls einem Cosolvens wie Methanol oder Ethanol eine wässrige, essigsaure, die Katalysatorkomponenten enthaltende Phase, die Katalysatorphase III. Diese Katalysatorphase III, die die Katalysatoren der Acylierung enthalten, wird mit der nach der Kondensation erhaltenen Katalysatorphase II vereinigt. Es resultiert eine wässrige, essigsaure Katalysatorphase, die quantitativ die aktiven Katalysatorkomponenten ZnX₂ enthält und einen Großteil der aktiven Katalysatorkomponente HY.

Diese Katalysatorphase wird durch entsprechende verfahrenstechnische Verfahren so behandelt, daß eine die Katalysatorkomponenten enthaltende Phase, die Katalysatorphase IV, resultiert, die nach Ergänzung der teilweise verbrauchten Komponente HY wieder für die Kondensation der Bausteine TMHQ-DA und Isophytol eingesetzt werden kann. Die Katalysator-Regenerierung umfasst im wesentlichen die teilweise Entfernung von Essigsäure und/oder Wasser, wobei im wesentlichen die Katalysatorkomponenten ZnX₂ und HY in einer konzentrierten Wasser/Essigsäure Lösung verbleiben. Im einfachsten Fall führt man zum diesem Zweck eine Destillation der vereinigten Katalysatorphasen II und III durch, wobei als Destillat Wasser und Essigsäure erhalten werden, ohne daß HY in Form einer konzentrierten, wässrigen Lösung im Destillat mit übergehen.

Die Destillation und die damit verbundene Regenerierung der Katalysatorphase wird bei einem Druck von 0,1 Torr bis 760 Torr durchgeführt. Die destillative Regenerierung der vereinigten Katalysatorphasen II und III wird entsprechend dem eingestellten Druck in einem Temperaturintervall von 20 °C - 200°C vollzogen. Die Möglichkeit, die Katalysator-Regenerierung bei reduziertem Druck und entsprechend moderaten Temperaturen durchzuführen, bietet zusätzliche Vorteile bezüglich der Wahl des Werkstoffes der verwendeten Apparate. In einer weiteren erfindungsgemäßen Variante wird die Katalysatorregenerierung durch Einengung der vereinigten Katalysatorphasen II und III so durchgeführt, daß neben Wasser und Essigsäure auch teilweise HY destillativ entfernt wird. Die resultierende Katalysatorphase IV muß dann zur Aufrechterhaltung der vollen katalytischen Aktivität mit der entsprechenden Konzentration an HY ergänzt werden.

Die Regenerierung der vereinigten Katalysatorphase kann neben den beschriebenen destillativen Methoden auch durch alternative Methoden erfolgen, insbesondere der Abtrennung von Wasser und /oder Essigsäure durch Separierung mittels einer geeigneten Membran. Nach dieser Variante erfolgt die Konzentrierung der aktiven Katalysatorlösung durch selektive Entfernung von Essigsäure und/oder Wasser, wobei ebenfalls eine Katalysatorlösung IV verbleibt, die neben einer, wie oben angegebenen Essigsäure-Wasser Konzentration, die aktiven Katalysatorkomponenten enthält. In einer weiteren Variation der Durchführung kann dem Extraktionsmedium (bestehend aus Wasser und einem Cosolvens wie Methanol oder Ethanol) 0,1 Gew.-% - 5 Gew.-% der entsprechenden Mineralsäure HY zugesetzt werden, um eine vollständige Extraktion der Katalysatorkomponenten aus der Produktphase III sicherzustellen.

Die nach der beschriebenen Vorgehensweise erhaltenen Katalysatorlösungen IV sind in einem Temperaturintervall von 0°C - 200°C auch beim mehrmaligen Recylieren ausreichend viskos, um sie mit geeigneten Pumpen im flüssigen Zustand zu fördern, ohne daß es es zur Kristallisation der Katalysatorkomponten kommt, die zusätzliche Maßnahmen zur Recyclierung erfordern.

Durch die erfindungsgemäße Durchführung der Kondensation von TMHQ-DA mit Isophytol in Essigsäure als Lösungsmittel und der beschriebenen Verfahrensweise zur Regenerierung der Katalysatorlösung als wässrige Essigsäure-enthaltende ZnX₂ - HY enthaltende Katalysatorlösung wird ein unkompliziertes, effizientes Verfahren zur direkten Herstellung von Vitamin E-Acetat bereitgestellt, das ohne oder mit nur unwesentlicher Ergänzung der Katalysatorkomponente HY eine gleichbleibende katalytische Aktivität des eingesetzten Katalysators ermöglicht.

Durch die erfindungsgemäße Durchführung der Vitamin E-Acetat Herstellung ex TMHQ-DA und Isophytol ist es gelungen, eine Lösungsmittel/Katalysatormatrix zu finden, die es ermöglicht, durch Verwendung eines wasserlöslichen, mit Wasser-extrahierbaren Lösungsmittels, insbesondere Essigsäure, eine selektive Produktherstellung nach Kondensation zu realisieren und eine Katalysatorabtrennung des Kondensationskatalysators von der erhaltenen Produktphase, bestehend aus Vitamin E/Vitamin E-Acetat und Essigsäure, ermöglicht, wobei nach der Katalysatorabtrennung von der Vitamin E/Vitamin E-Acetat Phase eine ausreichende Katalysatorkonzentration für die anschließende Acylierung mit einem geeigneten Acylierungsmittel bei moderaten Temperaturen ermöglicht wird und nach Acylierung unter Erhalt des Produkts Vitamin E-Acetat mit einem geeigneten Acylierungsmittel eine Extraktion der Katalysatorphase mit einem wässrigen Extraktionsmittel durchzuführen und durch Regenerierung der so erhaltenen Katalysatorphase unter Entfernung von Wasser/Essigsäure eine aktive, unter moderaten Temperaturen gut handhabbare, Katalysatorphase IV zu erhalten, die ohne Aktivitätsverlust für den wiederholten Einsatz als Katalysatorlösung verwendet werden kann.

Die folgenden Beispiele veranschaulichen das erfindungsgemäße Verfahren. Der Gehalt der nach Kondensation erhaltenen Mischungen wie der Gehalt der Produkte wurden nach Analyse der Produkte gegen verfügbare, marktgängige Präparate (Fluka: 98,5% Vit. E-Ac) quantifiziert.

### Beispiel 1

In einem 2 1 Vierhalskolben werden 112,6 g ZnBr₂, 300 ml (315 g) Eisessig und 50,6 g Bromwasserstoffsäure 48% vorgelegt und anschließend 300,1 g TMHQ-DA unter Rühren eingetragen. Nach kurzem Stickstoff-Spülen bei Raumtemperatur wird innerhalb von 10 Minuten auf 60°C aufgeheizt. Das Isophytol wird nun binnen 4 Stunden. bei 60°C zugegeben und im Anschluss 1 Stunde bei 60°C nachgerührt.

Nach Abkühlen auf Raumtemperatur werden die beiden Phasen getrennt und die Katalysatorphase I 2 mal mit 50 ml n-Hexan gewaschen. Die n-Hexan Extrakte werden mit der Produktphase I zur Produktphase II vereinigt. Zu dieser gibt man nun innerhalb von 45 Minuten eine mindestens stöchiometrische Menge Acetanhydrid so zu, sodass die Reaktionstemperatur 22°C nicht übersteigt und lässt im Anschluss noch 15 Minuten nachreagieren.

Danach gibt man zur schwarzen Reaktionslösung 350 ml n-Hexan und 250 ml Wasser und rührt ca. 10 Minuten kräftig durch. Die Emulsion wird im Scheidetrichter getrennt und die organische Phase 2 mal mit 50 ml Wasser gewaschen.

Die Produktphase IV wird am Rotationsverdampfer bei 60°C und 1 mbar bis zur Massekonstanz eingeengt. Das zurückgewonnene n-Hexan kann für nachfolgende Extraktionen wieder eingesetzt werden.

Die beiden Wasserextrakte (Katalysatorphase III) werden mit der Katalysatorphase II vereinigt und mittels einfacher Destillation, bestehend aus Liebig-Kühler mit Claisen-Aufsatz, bis zu einer Sumpftemperatur von 146°C eingeengt.

Man erhält dabei 179,5 g Rückstand (violette Lösung) der sich dadurch auszeichnet, dass er bei Raumtemperatur gut pumpbar und handhabbar ist.

Der Rückstand setzt sich wie folgt zusammen:
60 - 65 % ZnBr₂
10 - 13 % HBr
13 - 16 % Wasser
- 10 - 15 % AcOH

Dieser Rückstand kann ohne Ausbeute- und Selektivitätsverluste für die Folgeumsetzung wiederverwendet werden.

### Beispiel 2 - 9

Der aus Beispiel 1 erhaltene Rückstand wird mit den aus der Tabelle I ersichtlichen Mengen an ZnBr₂, HBr und AcOH versehen. Der für die Reaktion gewünschte Wassergehalt wurde durch die Zugabe von Ac₂O eingestellt, kann aber auch bei der Katalysatorrecyclierung durch die Abtrennung von Essig säure und Wasser reguliert werden. Es ist ersichtlich, daß auch bei achtmaliger Recyclierung der essigsauren Katalysatorphase kein Aktivitätsverlust eintritt. Die Ergebnisse sind in der tabelle I zusammengefasst.

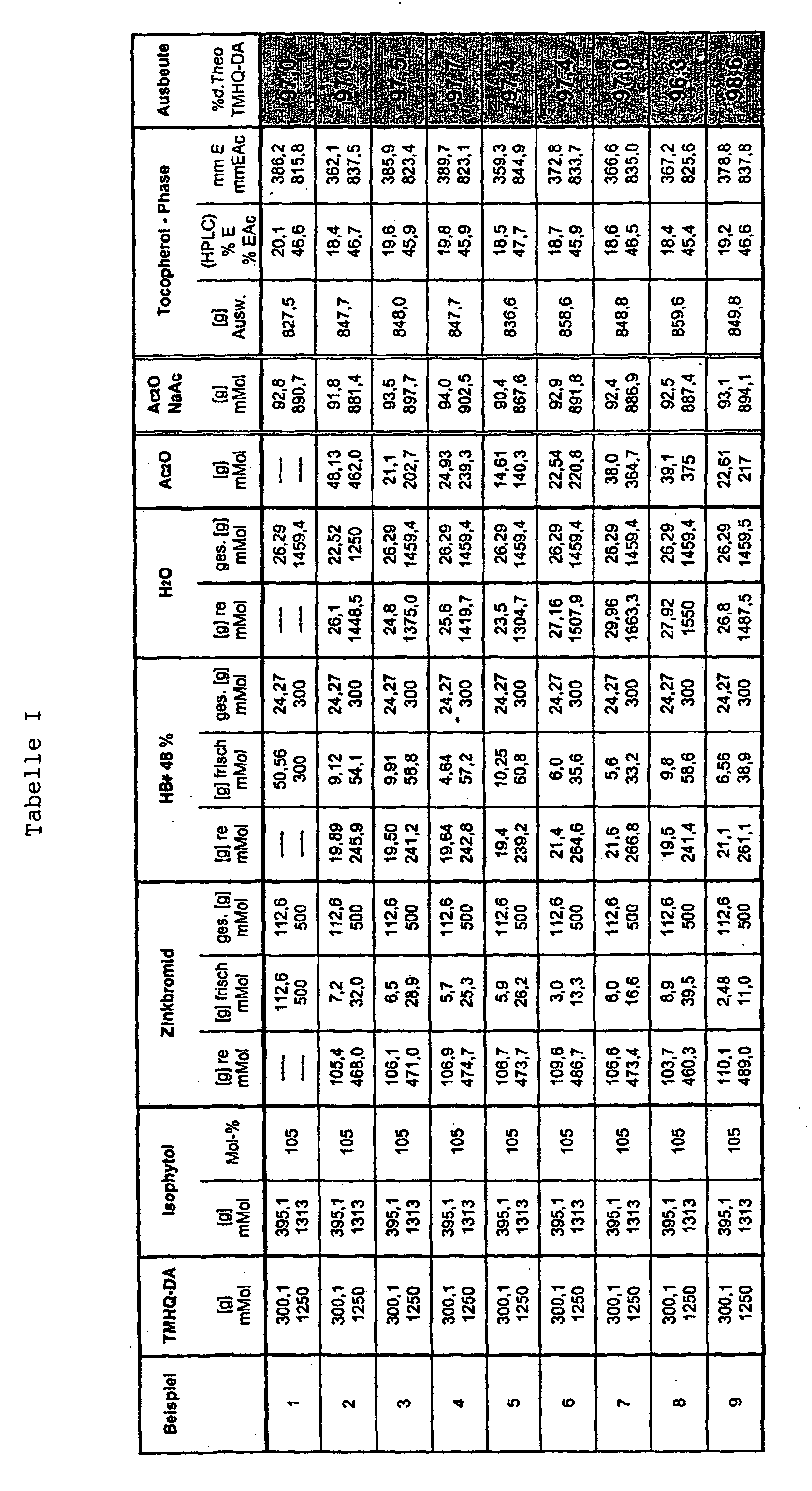

### Beispiel 10 - 12

Man verfährt analog Beispiel 1 und recykliert den Katalysator analog Beispiel 2 - 9, addiert jedoch mit dem jeweiligen TMHQ-DA zusätzlich noch 3,27 g Zn (4 Mol-% bez. auf TMHQ-DA) . Dies hat den Vorteil, dass man ein roh Vitamin E-Acetat mit grösserer Farbreinheit erhält und eine kostspielige ZnBr₂-Ergänzung umgangen werden kann und die Ergänzung fehlenden Zinkbromids durch die in situ Bildung ausgehend von wässriger HBr und Zn kompensiert werden kann.

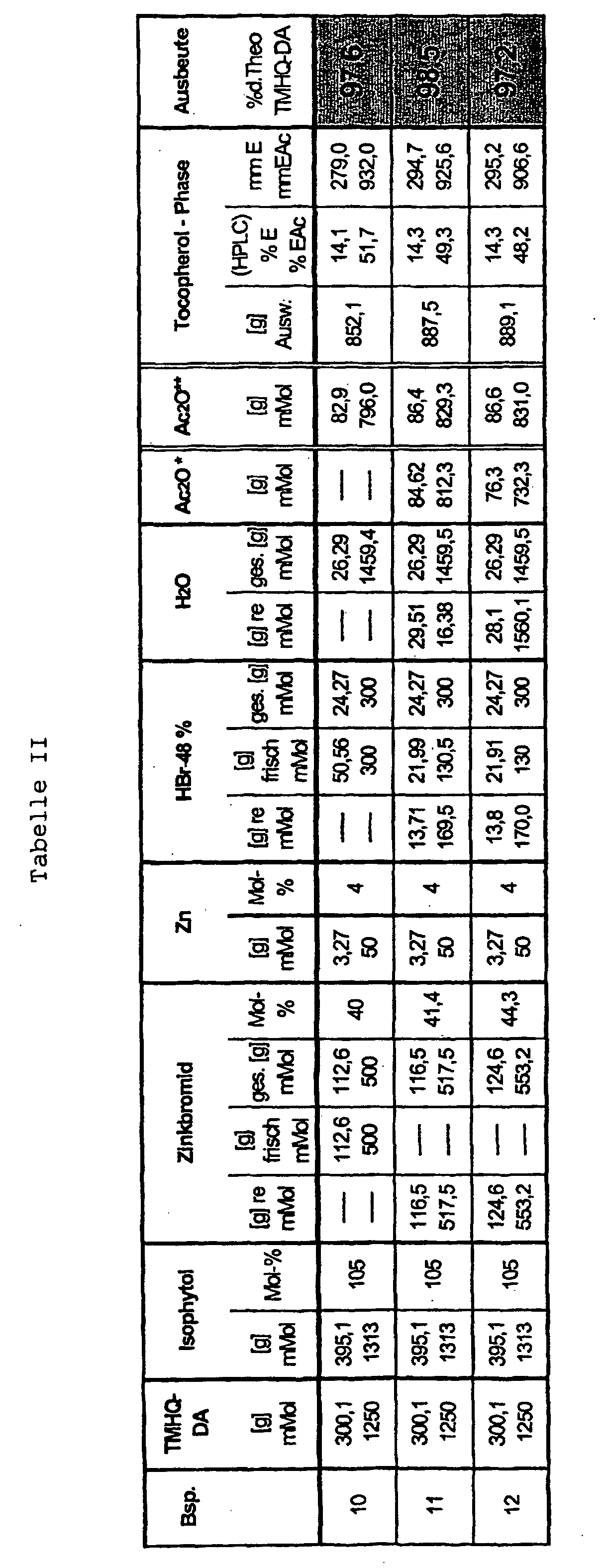

## Patentansprüche

1. Verfahren zur Herstellung von α-Tocopherol-Acetat im Kreislaufverfahren durch Kondensation von Trimethylhydrochinon-Diestern und Isophytol in Gegenwart eines Katalysatorsystems bestehend aus einem Zinkhalogenid und einer wässrigen Protonensäure und gegebenenfalls eines elementaren Metalls in einem mit Wasser extrahierbaren oder mischbaren polaren Lösungsmittel/Wasser Gemisch, **dadurch gekennzeichnet, dass** man
i) die Kondensation in Gegenwart von Essigsäure und Wasser als Lösungsmittel der Katalysatoren und Edukte durchführt,
ii) die zunächst erhaltene Mischung von α-Tocopherol und α-Tocopherolester mit Acetanhydrid unter Entstehung von Essigsäure verestert,
iii) die nach Aufarbeitung durch Extraktion anfallende essigsaure Lösung der Katalysatoren regeneriert und die regenerierte, Essigsäure enthaltende Katalysatorlösung in die Reaktion zurückführt, und
iv) die vereinigten, in i), ii) und iii) anfallenden essigsauren Katalysatorlösungen, die die aktiven Katalysatorkomponenten Zinkhalogenid/Protonensäure enthalten aufkonzentriert und in flüssiger Form in die Reaktion zurückführt.

2. Verfahren nach Anspruch 1, **dadurch gekennzeichnet, daß** als Zinkhalogenid Chlorid, Bromid, Oxy- und Hydroxychlorid sowie Oxy- und Hydroxy-bromid oder deren Gemische eingesetzt werden.

3. Verfahren nach Anspruch 1, **dadurch gekennzeichnet, daß** als Protonensäure Chlor- und Bromwasserstoffsäure eingesetzt wird und für das elementare Metall Zink steht.

4. Verfahren nach Anspruch 1, **dadurch gekennzeichnet, daß** als Extraktionsmittel für die Katalysatorlösung Essigsäure eingesetzt wird.

5. Verfahren nach Anspruch 1, **dadurch gekennzeichnet, daß** als Acylierungsmittel Essigsäureanhydrid eingesetzt wird.

6. Verfahren nach Anspruch 1, **dadurch gekennzeichnet, daß** man das wässrige essigsaure Katalysatorgemisch destillativ oder mittels Membranabtrennung aufkonzentriert.

7. Verfahren nach Anspruch 1, **dadurch gekennzeichnet, daß** die Reaktion im Kreislaufverfahren fortlaufend mehrmals durchgeführt wird.

## Claims

1. Process for the production of α-tocopherol acetate in a recycling process by condensation of trimethylhydroquinone diesters and isophytol in the presence of a catalyst system consisting of a zinc halide and of an aqueous protonic acid and optionally of an elemental metal in a polar solvent/water mixture which is extractable or miscible with water,
**characterized in that**
i) the condensation is carried out in the presence of acetic acid and water as solvents for the catalysts and educts,
ii) the initially obtained mixture of α-tocopherol and α-tocopherol ester is esterified with acetic anhydride with the formation of acetic acid.
iii) the acetic solution of the catalysts obtained after working up by extraction is regenerated and the regenerated solution containing acetic acid is returned to the reaction and
iv) the combined acetic catalyst solutions obtained in i), ii) and iii) containing the active catalyst components zinc halide/protonic acid are concentrated and in liquid form are returned to the reaction.

2. Process according to Claim 1,
**characterized in that**
the zinc halide used is chloride, bromide, oxy- and hydroxychloride as well as oxy- and hydroxybromide or mixtures thereof.

3. Process according to Claim 1,
**characterized in that**
the protonic acid used is hydrochloric acid and hydrobromic acid and the elemental metal is zinc.

4. Process according to Claim 1,
**characterized in that**
acetic acid is used as extracting agent for the catalyst solution.

5. Process according to Claim 1,
**characterized in that**
acetic anhydride is used as acylating agent.

6. Process according to Claim 1,
**characterized in that**
the aqueous acetic catalyst mixture is concentrated by distillation or by means of membrane separation.

7. Process according to Claim 1,
**characterized in that**
the reaction is carried out continuously and repeatedly in the recycling process.

## Revendications

1. Procédé pour la préparation d'acétate d'α-tocophérol dans un procédé en circulation par condensation de diesters de triméthylhydroquinone et d'isophytol en présence d'un système de catalyseur constitué d'un halogénure de zinc et d'un acide protonique aqueux et le cas échéant d'un métal élémentaire dans un mélange solvant polaire pouvant être extrait par l'eau ou miscible avec l'eau/eau, **caractérisé**
i) **en ce qu'**on réalise la condensation en présence d'acide acétique et d'eau comme solvant des catalyseurs et produits de départ,
ii) **en ce qu'**on estérifie le mélange d'abord obtenu d'α-tocophérol et d'ester d'α-tocophérol avec de l'anhydride de l'acide acétique avec formation d'acide acétique,
iii) **en ce que** régénère la solution d'acide acétique des catalyseurs formée après traitement par extraction et qu'on recycle la solution des catalyseurs, contenant de l'acide acétique dans la réaction et
iv) **en ce qu'**on concentre les solutions des catalyseurs contenant de l'acide acétique, formées dans i), ii) et iii), rassemblées, qui contiennent les composants actifs de catalyseur halogénure de zinc/acide protonique et qu'on les recycle sous forme liquide dans la réaction.

2. Procédé selon la revendication 1, **caractérisé en ce qu'**on utilise comme halogénure de zinc le chlorure, le bromure, l'oxychlorure et l'hydroxychlorure ainsi que l'oxybromure et l'hydroxybromure ou leurs mélanges.

3. Procédé selon la revendication 1, **caractérisé en ce qu'**on utilise comme acide protonique de l'acide chlorhydrique et de l'acide bromhydrique et que le métal élémentaire est du zinc.

4. Procédé selon la revendication 1, **caractérisé en ce qu'**on utilise de l'acide acétique comme agent d'extraction pour la solution de catalyseur.

5. Procédé selon la revendication 1, **caractérisé en ce qu'**on utilise de l'anhydride de l'acide acétique comme agent d'acylation.

6. Procédé selon la revendication 1, **caractérisé en ce qu'**on concentre le mélange de catalyseur aqueux d'acide acétique par distillation ou par séparation par membrane.

7. Procédé selon la revendication 1, **caractérisé en ce que** la réaction est réalisée en continu, plusieurs fois dans un procédé en circulation.
